Europäisches Patentamt

European Patent Office

Office européen des brevets

(19)

(11) Veröffentlichungsnummer : **0 033 110**
**B1**

# EUROPÄISCHE PATENTSCHRIFT

(12)

(45) Veröffentlichungstag der Patentschrift :
**13.03.85**

(51) Int. Cl.⁴ : **C 07 D301/16**

(21) Anmeldenummer : **81100353.2**

(22) Anmeldetag : **19.01.81**

(54) **Verfahren zur Herstellung von Epoxyalkanen.**

(30) Priorität : **26.01.80 DE 3002861**

(43) Veröffentlichungstag der Anmeldung :
**05.08.81 Patentblatt 81/31**

(45) Bekanntmachung des Hinweises auf die Patenterteilung : **13.03.85 Patentblatt 85/11**

(84) Benannte Vertragsstaaten :
**AT BE CH DE FR GB IT LI NL SE**

(56) Entgegenhaltungen :
**DD-A-  133 093**
**DE-A- 1 618 625**
**DE-A- 2 619 091**
**DE-A- 2 747 762**
**DE-B- 1 252 687**

(73) Patentinhaber : **Henkel Kommanditgesellschaft auf
Aktien
Postfach 1100 Henkelstrasse 67
D-4000 Düsseldorf-Holthausen (DE)
Degussa Aktiengesellschaft
Weissfrauenstrasse 9
D-6000 Frankfurt am Main 1 (DE)**

(72) Erfinder : **Lindemann, Manfred, Dr.
Eipassstrasse 83
D-5650 Solingen 19 (DE)**
Erfinder : **Weiss, Herbert, Dr.
Alarichstrasse 77
D-5000 Köln-Deutz (DE)**
Erfinder : **Schweizer, Dieter, Dr.
Achenbachstrasse 77
D-4000 Düsseldorf (DE)**
Erfinder : **Käbisch, Gerhard, Dr.
Palmstrasse 1
D-7888 Rheinfelden (DE)**
Erfinder : **Malitius, Horst
Blümleacker 5
D-7888 Rheinfelden (DE)**
Erfinder : **Raupach, Siegfried
Langentalstrasse 24
D-7888 Rheinfelden (DE)**
Erfinder : **Trübe, Rudolf
Ernst-Reuter-Strasse 24
D-7888 Rheinfelden (DE)**
Erfinder : **Wittmann, Hans
Katzenbuckelweg 12
D-7888 Rheinfelden (DE)**

## Beschreibung

Die Erfindung betrifft ein Verfahren zur Herstellung von Epoxyalkanen mit 11 bis 24 Kohlenstoffatomen und nichtendständigen Epoxygruppen durch Umsetzung entsprechender Olefine mit einem wasserhaltigen Gemisch aus Wasserstoffperoxid und Ameisensäure.

Aus der DE-AS 1 252 687 ist ein Verfahren bekannt, bei dem man Doppelbindungen enthaltende organische Verbindungen, insbesondere ungesättigte Fettsäuren und deren Esterderivate, mit wässrigen Gemischen aus Wasserstoffperoxid und Ameisensäure in Gegenwart von Anthrachinonverbindungen als Katalysatoren zu den entsprechenden Oxiranen epoxidiert. Die im Beispielteil der Druckschrift erwähnte Anwendung des Verfahrens auf längerkettige Olefine mit nicht näher spezifizierter Lage der Doppelbindungen führt zu einer so geringen Epoxidierungsausbeute, daß das Verfahren für eine technische Herstellung von Olefinepoxiden nicht geeignet erscheint. Die DE-OS 1 618 625 beschreibt ein Verfahren zur Herstellung von Oxiranen durch Umsetzung von olefinischen Doppelbindungen enthaltenden Verbindungen mit Perameisensäure in einem hydrophoben organischen Lösungsmittel. Die beschriebene Arbeitsweise stellt kein In-situ-Epoxidationsverfahren dar. Die Perameisensäure wird hier vielmehr aus einer wässrigen Lösung, in der sie sich aus Wasserstoffperoxid und Ameisensäure bildet, in eine Lösungsmittelphase hineinextrahiert, in der dann die Umsetzung mit den olefinischen Doppelbindungen stattfindet.

Durch die US-PS 2 485 160 ist ein Verfahren bekannt geworden, bei dem Ester höherer ungesättigter Fettsäuren wie Methyloleat, Octyloleat, Propylenglykoldioleat und Sojabohnenöl mit Perameisensäure, die in situ aus Ameisensäure und Wasserstoffperoxid gebildet wird, zu den entsprechenden Oxiranverbindungen epoxidiert werden. Dieses Verfahren blieb in seiner Anwendung offensichtlich auf die Epoxydation von olefinischen Verbindungen, die durch funktionelle Gruppen polarisiert sind, beschränkt. Nach Organic Reactions, Band VII, New York 1953, S. 383 wird Perameisensäure im allgemeinen nicht als Epoxydationsmittel angesehen, da die hohe Acidität der bei der Reaktion entstehenden und gegebenenfalls auch als Lösungsmittel vorhandenen Ameisensäure die meisten Oxiranringe sofort aufgehen läßt.

Es wurde nun gefunden, daß diese vorgefaßte Meinung für die Epoxydation von längerkettigen linearen Olefinen mit nichtendständigen Doppelbindungen nicht zutrifft und daß man diese Verbindungen sehr wohl mit in situ aus Ameisensäure und Wasserstoffperoxid gebildeter Perameisensäure epoxydieren kann.

Gegenstand der Erfindung ist ein Verfahren zur Herstellung von Epoxyalkanen mit 11 bis 24 Kohlenstoffatomen aus geradkettigen Olefinen mit vorwiegend nichtendständigen Doppelbindungen durch Epoxydation mit einem wasserhaltigen Gemisch aus Wasserstoffperoxid und einer Carbonsäure. Dieses Verfahren ist dadurch gekennzeichnet, daß pro Mol zu epoxidierender Doppelbindung 0,05 bis 0,5 Mol Ameisensäure und 1,1 bis 2 Mol Wasserstoffperoxid eingesetzt werden und die Reaktion in Abwesenheit von Katalysatoren und organischen Lösungsmitteln bei 40 bis 70 °C durchgeführt wird.

Die erfindungsgemäße Herstellung von Epoxyalkanen geht von Olefinen mit 11 bis 24 Kohlenstoffatomen und nichtendständigen Doppelbindungen aus. In der Regel liegen solche Olefine nicht als chemische Individuen mit bestimmter Kettenlänge und festgelegter Position der innenständigen Doppelbindung vor, sondern als Gemische von Verbindungen mit unterschiedlicher Verteilung der Doppelbindung auf die an der Kohlenwasserstoffkette vorhandenen Innenpositionen. Solche Olefingemische können beispielsweise durch katalytische Dehydrierung oder Chlorierung-Dehydrochlorierung von Paraffinen mit 11 bis 24 Kohlenstoffatomen und selektive Extraktion der innenständigen Monoolefine erhalten werden. Weiterhin können solche Olefingemische mit nichtendständigen Doppelbindungen durch Isomerisierung von alpha-Olefinen, beispielsweise solchen aus der Äthylenpolymerisation, hergestellt werden. Die Isomerisierung kann in der Dampfphase erfolgen, wobei Katalysatoren wie Aluminiumoxid, Chromoxid-Aluminiumoxid und Magnesiumoxid-Aluminiumoxid zur Anwendung kommen. Die Isomerisierung der alpha-Olefine kann auch in der flüssigen Phase durchgeführt werden, wobei Katalysatoren wie Natrium-Aluminiumoxid und Kaliumaluminiumoxid und bestimmte Sulfonsäuregruppen enthaltende Ionenaustauscherharze verwendet werden. Die als Ausgangsmaterial eingesetzten Olefingemische können, bedingt durch ihre Herstellung einen geringen Anteil an verzweigtkettigen Verbindungen enthalten.

Bei der Herstellung von Epoxyalkanen nach dem erfindungsgemäßen Verfahren werden bevorzugt Fraktionen von Monoolefinen mit nichtendständigen Doppelbindungen mit einem hohen Gehalt an linearen Olefinen der Kettenlänge $C_{11}$-$C_{18}$ eingesetzt, beispielsweise $C_{11}$-$C_{14}$- und -$C_{15}$-$C_{18}$-Olefinfraktionen der nachstehend angegebenen prozentualen Verteilung der Kettenlänge :

a) Fraktion $C_{11}$-$C_{14}$

| | |
|---|---|
| $C_{11}$-Olefine | ca. 22 Gew.-% |
| $C_{12}$-Olefine | ca. 30 Gew.-% |
| $C_{13}$-Olefine | ca. 26 Gew.-% |
| $C_{14}$-Olefine | ca. 22 Gew.-% |

b) Fraktion $C_{15}$-$C_{18}$

| | |
|---|---|
| $C_{15}$-Olefine | ca. 26 Gew.-% |
| $C_{16}$-Olefine | ca. 35 Gew.-% |
| $C_{17}$-Olefine | ca. 31 Gew.-% |
| $C_{18}$-Olefine | ca. 6 Gew.-% |

Die für das erfindungsgemäße Verfahren erforderliche Ameisensäure kommt normalerweise in Form von wässrigen Lösungen mit höchstens 30 Gew.-% Wasser zum Einsatz. Wasserstoffperoxid wird in Form von wässrigen Lösungen mit einem Wasserstoffperoxidgehalt von 60 bis 92 Gew.-% eingesetzt.

Die Ameisensäure kann in Mengen von 0,05 bis 0,5 Mol pro Mol zu epoxidierender Doppelbindung eingesetzt werden, wobei Mengen von 0,1 bis 0,2 Mol Ameisensäure pro Mol zu epoxidierender Doppelbindung bevorzugt werden.

Die Verwendung von Lösungsmitteln und Katalysatoren ist in dem erfindungsgemäßen Verfahren nicht erforderlich. Die Anwesenheit von sauren Katalysatoren, wie sie bei der in situ-Epoxydation mit Essigsäure üblich sind, hat sich wegen der Bildung von unerwünschten Nebenprodukten als nachteilig erwiesen.

Bei der praktischen Durchführung des erfindungsgemäßen Verfahrens verfährt man zweckmäßigerweise so, daß man das zu epoxidierende Olefingemisch zusammen mit der gesamten Menge Ameisensäure vorlegt und das Gemisch auf die vorgesehene Reaktionstemperatur erwärmt. Anschließend wird dann die vorgesehene Menge Wasserstoffperoxidlösung unter Rühren langsam zugegeben. Die bei der Umsetzung entstehende Wärme muß durch Kühlen abgeführt werden, wenn die Wasserstoffperoxidzugabe in wirtschaftlich annehmbaren Zeiträumen vor sich gehen soll. Nach beendeter Zugabe wird das Reaktionsgemisch zur Vervollständigung der Reaktion 5 bis 10 Stunden lang weitergerührt, wobei man zweckmäßigerweise die gewählte Reaktionstemperatur aufrecht erhält.

Zur Isolierung der Reaktionsprodukte läßt man das Reaktionsgemisch stehen, bis die Phasentrennung vollständig ist. Die wässrige Schicht wird abgetrennt. Die organische Phase wird mit Wasser gewaschen, bis mindestens ein pH-Wert von 4 erreicht ist. Zur Entfernung von noch vorhandenen sauren Anteilen werden die Reaktionsprodukte zweckmäßigerweise bei erhöhter Temperatur mit verdünnter Natrium- oder Kaliumhydroxidlösung behandelt. Nach dem Abtrennen der alkalischen, wässrigen Lösung werden die Produkte bei erhöhter Temperatur unter vermindertem Druck getrocknet. Zur weiteren Reinigung können die erhaltenen Epoxyalkangemische unter Verwendung von Filterhilfen filtriert werden.

Die nach dem erfindungsgemäßen Verfahren hergestellten Epoxyalkangemische weisen einen Epoxidsauerstoffgehalt von 90 bis nahezu 100 % auf und sind im allgemeinen nur durch Spuren von nicht umgesetzten Olefinen und sekundären Reaktionsprodukten verunreinigt. Sie können praktisch für alle Synthesen oder direkten Anwendungen ohne weitere Reinigungsoperationen eingesetzt werden.

Die nachfolgenden Beispiele sollen die Erfindung erläutern, ohne sie hierauf zu beschränken.

## Beispiele

### Beispiel 1

Ein Gemisch aus 96 kg (541 Mol) einer Olefinfraktion der Kettenlänge $C_{11}$-$C_{14}$ mit nichtendständigen Doppelbindungen (Jodzahl 143) und 4,6 kg (85 Mol) 85-gewichtsprozentige Ameisensäure wurde in einem Rührgefäß mit Heiz- und Kühleinrichtung unter Rühren auf 55 °C erwärmt. Im Verlauf von 2 Stunden wurden sodann 34 kg (700 Mol) 70-gewichtsprozentige Wasserstoffperoxidlösung zugegeben. Durch die alsbald einsetzende exotherme Reaktion stieg die Temperatur im Rührbehälter an. Durch Kühlen wurde eine Reaktionstemperatur von etwa 60 °C einreguliert. Nach dem Ende der Wasserstoffperoxidzugabe wurde das Reaktionsgemisch 8 Stunden bei 60 °C weitergerührt. Beim Stehenlassen bildete das Reaktionsgemisch zwei Schichten. Die wässrige Schicht wurde abgetrennt und verworfen. Die organische Schicht wurde mit 400 l heißem Wasser bis zum pH-Wert 4 gewaschen. Anschließend wurde das Reaktionsprodukt mit 12 l 2-gewichtsprozentiger Kaliumhydroxidlösung bei 80 bis 90 °C raffiniert. Nach der Raffination wurde das Produkt mit 500 l heißem Wasser bis zur neutralen Reaktion gewaschen, durch Erhitzen auf 90 °C bei 40 mbar getrocknet und filtriert. Es wurden 100 kg Epoxyalkangemisch mit einem Epoxidsauerstoffgehalt von 7,5 Gewichtsprozent (90,5 % des theoretischen Gehaltes) und der Rest-Jodzahl 5,1 erhalten.

### Beispiel 2

Ein Gemisch aus 47 kg (197,5 Mol) einer Olefinfraktion der Kettenlänge $C_{15}$-$C_{18}$ mit nichtendständigen Doppelbindungen (Jodzahl 105) und 1,6 kg (29,6 Mol) 85-gewichtsprozentiger Ameisensäure wurde in einem Rührgefäß mit innenliegender Heiz- und Kühleinrichtung unter Rühren auf 50 °C erwärmt. Im Verlauf von einer Stunde wurden 12,5 kg (257 Mol) 70-gewichtsprozentige Wasserstoffperoxidlösung

# 0 033 110

langsam zugegeben, wobei die Temperatur des Gemisches durch Kühlen auf etwa 60 °C einreguliert wurde. Anschließend wurde das Reaktionsgemisch 9 Stunden bei dieser Temperatur weitergerührt. Nach dem Abtrennen der wässrigen Phase wurde die organische Phase wie in Beispiel 1 beschrieben aufgearbeitet. Es wurden 48,5 kg Epoxyalkangemisch mit 5,73 Gewichtsprozent Epoxidsauerstoffgehalt (92,4 % des theoretischen Gehaltes) und der Jodzahl 4,3 erhalten.

### Beispiel 3

Ein Gemisch aus 109 kg (700 Mol) einer Olefinfraktion der Kettenlänge $C_{11}$-$C_{12}$ mit nichtendständigen Doppelbindungen (Jodzahl 163) und 7,6 kg (140 Mol) 85-gewichtsprozentiger Ameisensäure wurde unter Rühren auf 50 °C erwärmt. 41,7 kg (981 Mol) 80-gewichtsprozentiges Wasserstoffperoxid wurden im Verlauf von 90 Minuten zugegeben. Während der Zugabe wurde die Temperatur des Gemisches durch Kühlen auf 60 °C einreguliert. Nach dem Ende der Wasserstoffperoxidzugabe wurde das Gemisch 10 Stunden lang bei 60 °C weitergerührt. Nach dem Abtrennen der wässrigen Schicht wurde die organische Phase zweimal mit Kondenswasser gewaschen und anschließend bei 80 bis 90 °C mit 12 l 2-prozentiger Natriumhydroxidlösung behandelt. Das Produkt wurde noch einmal durch Aufsprühen von 50 l Wasser gewaschen, bei 90 °C im Ringpumpenvakuum bei 40 mbar getrocknet und schließlich über Celite als Filtrierhilfsmittel filtriert. Es wurden 120 kg farbloses Epoxyalkangemisch mit einem Epoxidsauerstoffgehalt von 8,57 Gew.-% (92 % des theoretischen Gehaltes) und der Jodzahl von 2,35 erhalten.

### Beispiel 4

Zu einem auf 60 °C erwärmten Gemisch aus 113 kg (598 Mol) einer Olefinfraktion der Kettenlänge $C_{13}$-$C_{14}$ mit nichtendständigen Doppelbindungen (Jodzahl 138,5) und 3,25 (60 Mol) 85-gewichtsprozentiger Ameisensäure wurden im Verlauf von 90 Minuten unter Rühren 43,7 kg (900 Mol) 70-gewichtsprozentige Wasserstoffperoxidlösung zugegeben. Dabei wurde die Temperatur des Gemisches durch Kühlen auf 60 °C gehalten. Anschließend wurde das Gemisch 8 Stunden lang bei 60 °C weitergerührt. Die Aufarbeitung des erhaltenen Reaktionsgemisches erfolgte analog Beispiel 1. Es wurden 118 kg Epoxyalkangemisch mit einem Epoxidsauerstoffgehalt von 7,56 Gew.-% (94,2 % des theoretischen Gehaltes) erhalten. Die Jodzahl des Produktes betrug 2,57.

### Ansprüche

1. Verfahren zur Herstellung von Epoxyalkanen mit 11 bis 24 Kohlenstoffatomen aus geradkettigen Olefinen mit nichtendständigen Doppelbindungen durch Epoxydation mit einem wasserhaltigen Gemisch aus Wasserstoffperoxid und einer Carbonsäure, dadurch gekennzeichnet, daß pro Mol zu epoxidierender Doppelbindung 0,05 bis 0,5 Mol Ameisensäure und 1,1 bis 2 Mol Wasserstoffperoxid eingesetzt und die Reaktion in Abwesenheit von Katalysatoren und organischen Lösungsmitteln bei 40 bis 70 °C durchgeführt wird.

2. Verfahren nach Anspruch 1, dadurch gekennzeichnet, daß man Olefine mit 11 bis 18 Kohlenstoffatomen epoxidiert.

3. Verfahren nach den Ansprüchen 1 und 2, dadurch gekennzeichnet, daß pro Mol zu epoxidierender Doppelbindung 0,1 bis 0,2 Mol Ameisensäure eingesetzt werden.

### Claims

1. A process for the production of epoxyalkanes containing from 11 to 24 carbon atoms from straight-chain olefins containing non-terminal double bonds by epoxidation with an aqueous mixture of hydrogen peroxide and a carboxylic acid, characterized in that from 0.05 to 0.5 mole of formic acid and from 1.1 to 2 moles of hydrogen peroxide are used per mole of double bond to be epoxidized and the reaction is carried out at 40 to 70 °C in the absence of catalysts and organic solvents.

2. A process as claimed in Claim 1, characterized in that olefins containing from 11 to 18 carbon atoms are epoxidized.

3. A process as claimed in Claims 1 and 2, characterized in that from 0.1 to 0.2 mole of formic acid is used per mole of double bond to be epoxidized.

### Revendications

1. Procédé pour la fabrication d'époxyalcanes possédant 11 à 24 atomes de carbone, à partir d'oléfines à chaîne linéaire comportant des doubles liaisons non terminales, par époxydation avec un mélange contenant de l'eau oxygénée et un acide carboxylique, procédé caractérisé en ce que l'on met en œuvre, par molécule dont la double liaison doit être oxydée, 0,05 mol à 0,5 mol d'acide formique et 1,1

4

à 2 mol de peroxyde d'hydrogène, et on effectue la réaction à 40 à 70 °C, sans catalyseur ni solvants organiques.

2. Procédé suivant la revendication 1, caractérisé en ce que l'on époxyde des oléfines à 11 à 18 atomes de carbone.

3. Procédé suivant l'une des revendications 1 et 2, caractérisé en ce que l'on met en œuvre 0,1 à 0,2 mol d'acide formique par mol à double liaison que l'on doit oxyder.